# EUROPEAN PATENT APPLICATION

(11) **EP 4 248 750 A1**
(43) Date of publication of application: **27.09.2023**
(21) Application number: 20960986.6
(22) Date of filing: 13.11.2020
(51) Int. Cl.: A01N 63/00, A01N 63/20, A01N 63/22, C12R 1/01, C12R 1/07, C12R 1/425, C12R 1/645

(54) **FREEZE-DRIED BACTERIAL CONSORTIUM FOR CONTROLLING GAEUMANNOMYCES GRAMINIS**

(71) Applicant: Universidad De La Frontera, Temuco, casilla 54-D (CL)
(72) Inventor: DURÁN CUEVAS, Paola Andrea, Santa Olga (CL); MERIÑO GERGICHEVICH, Cristian Jorge, Freire Temuco (CL); BARRA ESPINOZA, Patricio Javier, 01145 Temuco (CL); VISCARDI, Sharon, Padre Las Casas Temuco (CL); MÉNDEZ PANES, Isabel Audelia, 0802 Temuco (CL)
(74) Representative: Clarke, Modet y Cía., S.L.
(86) International application number: PCT/CL2020/050152
(87) International publication number: WO 2022/099427

(57) **Abstract**

Disclosed are: a freeze-dried bacterial consortium comprising a strain of Acinetobacter sp., a strain of Serratia sp. and a strain of Bacillus sp.; a method for preparing same; and a use and method applying same, to inhibit the development of diseases caused by plant pathogens and to promote plant growth.

## Description

### Field of the invention

The present invention discloses a lyophilized bacterial consortium comprising two endophytic bacterial strains (*Acinetobacter sp* and *Serratia sp*) and a rhizospheric bacterial strain (*Bacillus sp*)*,* the method of obtaining said consortium, and the use of said consortium to inhibit the development prevent and/or treat diseases caused by plant pathogens and to promote plant growth. The lyophilized bacterial consortium is applied to plant seeds to inhibit the development of diseases caused by plant pathogens, as well as to promote growth in plants.

### Problem of the technique

The disease "take-all" is a disease caused by the fungus *Gaeumannomyces graminis* var. *Tritici (Ggt).* This phytopathogen affects the roots of cereals such as wheat, rye and triticale, causing significant economic losses. In the case of wheat, which is the most economically and culturally important cereal, production losses caused by this fungus range from 30% to 50%, this being one of the most severe diseases that affects crops worldwide. world. Added to this, the ability of the fungus to form survival structures, such as apothecia, allows it to survive in the soil or in tissue remains for a long time, which makes control extremely difficult and is mainly restricted to the use of chemical agents to treat this disease. In this regard, the disinfection of soil or seed through the use of fungicides such as azoxystrobin, silthiofam and triticonazole stands out. However, the use of chemical agents generates an extra economic expense, which is accompanied by the corresponding environmental contamination and the ecological damage caused to the ecosystem that their use generates.

On the other hand, and in order to limit contamination due to chemical agents, other agricultural practices or alternative planting systems have been used, wherein crop rotations stand out, which aim at not planting the same family of plants for periods of time in a row. Under this strategy, the more years elapse between one planting of a certain crop and the next, the greater the control against the disease. However, as this phytopathogen affects other cereals, farmers are forced to carry out crop rotations discarding other grass species to avoid or reduce the risk of subsequent infections, with which their crop options are severely restricted.

Additionally, and given the complexity of carrying out adequate rotations for the control of the disease caused by *Ggt*, and in search of new alternatives to combat this pathogen, research has been carried out that has shown that sowing cereals consecutively in the same place, a process called monoculture, has led to the development of disease-suppressive soils. Suppressive soils are defined as those soils that, despite the presence of the pathogen, are immune or have a low incidence of the disease, mainly due to the presence of beneficial microorganisms that help control the phytopathogen (Duran *et al.,* 2017. Screening and characterization of potentially suppressive soils against *Gaeumannomyces graminis* under extensive wheat cropping by *Chilean indigenous communities).* Among these microorganisms, it is possible to highlight the plant growth-promoting bacteria or PGPB that are capable of helping plants survive environmental factors (abiotic) or diseases caused by phytopathogens (biotic factors) by producing enzymes, hormones or antibiotics.

Within the production of antibiotics is the production of phenazine, which is exclusively produced by bacteria. However, and despite the numerous studies regarding the use of PGPB for agronomic applications, its use at the field level has been very limited. This is due, among other reasons, mainly to the fact that foreign microorganisms often have a low persistence and permanence in the soil where they are introduced due to the arduous competition with native microorganisms. Additionally, the formulations that have been tested in the field are usually very unstable and do not ensure the activity of microorganisms, so the formulation that contains the microorganisms, and consequently the method by which it is produced, is crucial to ensure stability, survival and activity of PGPB. A possible solution to this problem is the use of microbial consortia formulated with endophytic bacteria, which are PGPB bacteria capable of surviving inside plant tissues without causing damage to it, which brings multiple evolutionary advantages. In this way, the bacteria colonize a more benevolent environment inside the plant, which favors its survival and consequently its positive effects. However, there is no practical evidence of the use of PGPB for the control of take-all disease caused by G*gt*.

Consequently, considering the technical problem associated with the take-all caused by *Ggt,* added to the fact that there is no effective strategy, friendly to the environment and that also allows the cultivation of grass species continuously without the risk of crops contracting and spreading this phytopathogenic fungus to future generations, it is of crucial importance to seek new strategies and alternatives to solve said technical problem.

### State of the art

In addition to the control through chemical agents such as the aforementioned fungicides, in an attempt to effectively control the fungus *Gaeumannomyces graminis* var. *Tritici (Ggt),* various strategies have been developed with a view to developing more natural and/or environmentally friendly alternatives.

In this way, there are currently treatments where the use of seed coatings is demonstrated to promote the health and growth of a plant. Thus, for example, patent application US 20170245494 discloses a bioactive and biodegradable multilayer film. The multilayer film includes one or more bioactive compounds or microorganisms to promote plant growth and health which are contained between the layers of the film, each layer comprising about 60% to about 75% (w/w) of polyhydroxyalkanoate. Bioactive compounds or microorganisms can include any of, or a combination of: a metabolite, an antimicrobial compound, an enzyme, a living microorganism, a fertilizer, a plant growth hormone, a preservative, a pesticide, or an herbicide. The release of one or more bioactive compounds can be achieved in a controlled and programmed manner. It should be noted that document US 20170245494 does not disclose a bacterial consortium lyophilized as disclosed in this application. Additionally, the present invention differs from document US 20170245494, in that the microorganisms used by the inventors are bacteria isolated from soils with suppressive characteristics, which are capable of producing phenazine-type antibiotics, and are also capable of promoting the growth of plants by producing enzymes that solubilize phosphorus (such as phosphatases and phytases), making it available for plant nutrition. Along with the above, the bacteria of the consortium of the present invention also produce siderophores (such as hydroxamates), which are capable of chelating iron and making it available to the plant, which indirectly prevents it from being available for the phytopathogen nutrition. Therefore, the present invention brings together several technical elements that document US 20170245494 does not disclose or suggest and that, therefore, substantially differentiate it from said document.

On the other hand, isolated microbial consortia have been used with the ability to generate molecules capable of promoting plant growth, as well as resistance to diseases. In this field, document CN 106244490 discloses a microbial fertilizing compound for preventing and treating plant diseases, particularly wheat, and a method of preparing the microbial fertilizing compound. The microbial fertilizer compound consists of the following strains: *Streptomyces catenulae, Streptomyces albolongus, Bacillus flexus, Bacillus aryabhattai* and *Trichoderma brevicompactum.* The strains in the document induce the wheat plant to generate resistance to diseases by virtue of the production of secondary metabolites and of various antibiotics, such as proceomycin, neomycin E, catenulin, diparomomycin and the like, which inhibit disease pathogenic microorganisms of wheat. The five strains selected in the document CN 106244490 were directly isolated from the soil and have a promoting effect on the growth of the rhizosphere. In addition, they can promote reproduction and growth on growing surfaces or in plants or soil. Additionally, document CN 106244490 discloses that the strains can have an effect on nitrogen fixation and can promote the directional secretion of some secondary metabolites from the rhizosphere of the plant.

Likewise, the use of individual bacterial strains to treat take-all disease has also been reported. Thus, for example, document CN 103074271 discloses a pulverized microbial inoculum, prepared by preparing a dry aerosol fermentation solution of the strain of *Bacillus subtilis* YB-05. According to the document, the YB-05 strain and its inoculant would have the advantages of having a broad antibacterial spectrum, good antimicrobial effect, and strong inhibition effects on wheat disease (take-all), *Botrytis cinerea* on tomato, wheat sheath blight fungi, *Phytophthora melonis* and the like. Furthermore, the inoculant would have the advantages of high efficiency, non-toxicity, simple use and no environmental pollution.

It is important to note that the aforementioned documents CN 106244490 and CN 103074271 do not disclose a bacterial consortium lyophilized as disclosed in this application. In addition, unlike the microorganisms disclosed in these documents, the bacterial consortium lyophilized of the present invention was isolated from suppressive soils. Thus, for example, *Serratia* sp corresponds to a microorganism isolated from the tissues of plants that were grown in suppressive soils (endophytic strain), *Bacillus sp.* corresponds to a microorganism isolated from the soil in contact with the roots of the plants (rhizospheric strain), and finally *Acinetobacter sp.* was also isolated from the inside of wheat roots (endophytic strain). In this context, it should be noted that the use of endophytic microorganisms has been shown to provide a comparative advantage mainly because the microorganisms grown in these environments are microorganisms that have evolved to generate an association between the microorganisms and the plant. This evolutionary advantage helps promote a beneficial effect, reflected in protection against abiotic factors (drought, salinity, pH, nutrients, etc.) and biotic factors (such as competition with other microorganisms). Therefore, microorganisms isolated from suppressive soils, such as those of the present invention, are significantly different from the microorganisms of the documents CN 106244490 and CN 10307427. Along with the aforementioned differences, although both documents describe that the strains would produce antibiotics, those strains are not described as producing phenazines, antibiotics being produced by the consortium of the present invention. Additionally, the bacteria used in the present invention also produce siderophores, such as hydroxamates, which are capable of chelating iron and making it available to the plant, which indirectly prevents it from being available for nutrition by the phytopathogen. Said technical characteristic associated with the production of siderophores was not described or suggested in any of the documents CN 106244490 and CN 10307427. Therefore, the present invention brings together several technical elements that substantially differentiate it from said documents of Chinese origin.

On the other hand, the state of the art discloses the use of microorganisms, as individual strains, together with phenazine-type antibiotics to treat plant diseases. In this context, for example, document CN 107306999 discloses a bactericidal composition based on phenazine-1-carboxylic acid and a bacterium that contains it, where the active components of the bactericidal composition are phenazine-1-carboxylic acid and *Bacillus cereus,* which are found in a weight ratio of phenazine-1-carboxylic acid to *Bacillus cereus* of 1:100-100:1, where the content of *Bacillus cereus* per gram of bactericidal composition is 10⁶ - 10¹¹/g, and the bactericide contains 1-90% by weight of the bactericidal composition. In contrast to this document, the present invention is based on a bacterial consortium and not on an individual strain, where, as previously highlighted, the bacterial consortium is made up of bacteria isolated from suppressive soils, with all the advantages that this entail. Likewise, in the document of Chinese origin, they use a combination of the microorganism *Bacillus cereus* with a type of phenazine, particularly phenazine-1-carboxylic acid, which according to the application is produced by *Pseudomonas fluorescens,* therefore, the effect of the microorganism and the metabolites it produces is not tested. Similarly, it is not described or suggested that the strain of *Bacillus cereus* produce enzymes capable of solubilizing phosphorus, nor siderophores. Additionally, the particular use to inhibit the development of the pathogen or to control the "take-all" disease of the wheat caused by *Ggt.*

Therefore, the present invention differs from the cited documents, in that it discloses a bacterial consortium lyophilized not described in the prior art, which in addition to producing the phenazine antibiotic, also provides transcendental factors for plant nutrition and health through, for example, the solubilization of phosphorus and the production of siderophores. In this context, the invention of the present patent application is based particularly on a bacterial consortium lyophilized consisting of two bacteria isolated from suppressive soils, *Bacillus sp.* (deposited in the Chilean Collection of Microbial Genetic Resources (CChRGM), under the Access No.: RGM 2948, dated January 28, 2020) and *Serratia sp.* (deposited in the CChRGM, under the Access No.: RGM 2949, dated January 28, 2020) and a bacterium called *Acinetobacter sp.* isolated from preliminary studies (Duran *et al.,* 2014, *Endophytic bacteria from selenium-supplemented wheat plants could be useful for plant-growth promotion, biofortification and Gaeumannomyces graminis biocontrol in wheat production;* deposited in the CChRGM, under the Access No.: RGM 3033, dated August 11, 2020). The use of these evolved bacteria in suppressive soils ensure control over plant pathogens, as well as help promote their growth and health.

Consequently, from the documents of the state of the art it is possible to conclude that to date there is no bacterial consortium lyophilized such as the one disclosed in the present invention that solves the problem of the technique, corresponding to treat diseases caused by plant pathogens (such as wheat, rye, triticale, vegetables and crops), more particularly the "take-all" disease caused by the phytopathogenic fungus *Gaeumannomyces graminis* (*Ggt*), without affecting, or even promoting, growth in plants. Therefore, specifically, the prior art does not disclose or suggest the bacterial consortium lyophilized of the present application, capable of not only reducing the percentage of infection of the pathogen *Gaeumannomyces graminis* which causes take-all in cereals, but also promotes the health of the plant through the solubilization of phosphorus and production of siderophores.

### Description of the figures

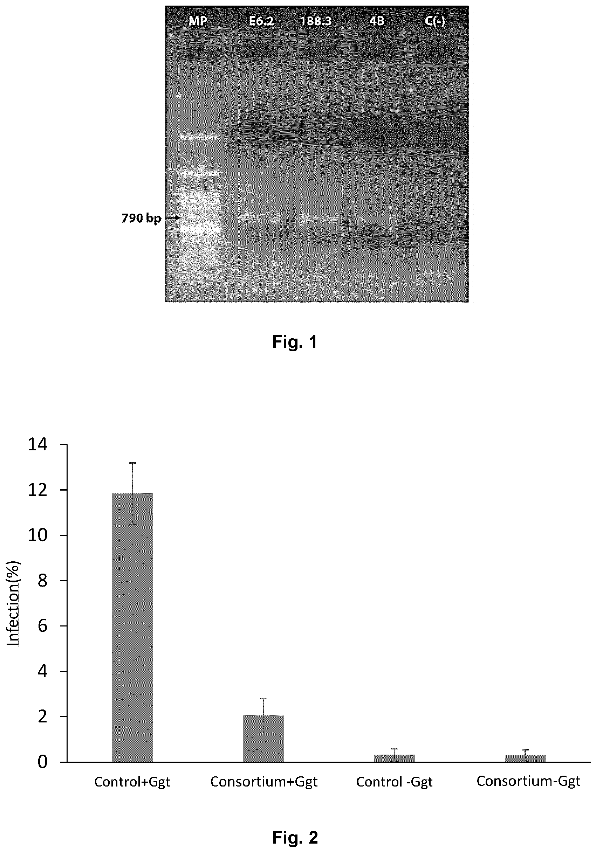

Figure 1: This figure shows a photograph of an agarose gel where a PCR amplification is observed where the expected size for the gene that codes for phenazine (790 bp) is observed. 1% agarose gel nomenclature, MP: molecular weight marker; E6.2: *Acinetobacter sp.* E6.2 (Access No.: RGM 3033); 188.3: *Serratia sp.* 188.3 (Access No.: RGM 2949); 4B: *Bacillus sp.* 4B (Access No.: RGM 2948); C(-): negative control.
Figure 2: This figure shows a graph where the percentage of infection is compared in wheat plants, grown from seeds inoculated with the lyophilized bacterial consortium (consortium), or without the lyophilized bacterial consortium (control), sown in soils in the presence of the fungus *Gaeumannomyces graminis* var. *Tritici (Ggt)* (+Ggt) or in the absence of *Ggt* (-Ggt).

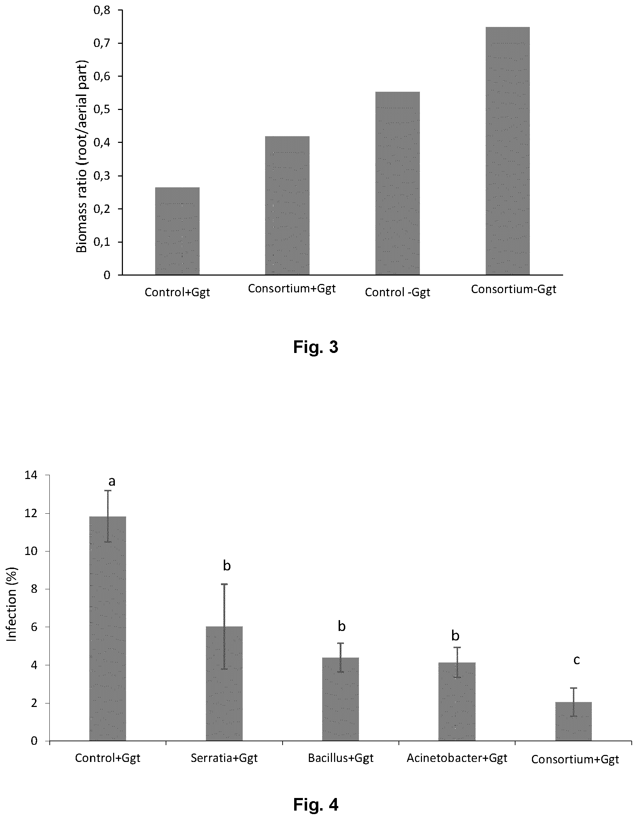

Figure 3: This figure shows a graph comparing the biomass obtained from wheat plants grown from seeds inoculated with the lyophilized bacterial consortium (consortium), or without the lyophilized bacterial consortium (control), sown in soils in the presence of the Ggt fungus (+Ggt) or in the absence of *Ggt* (-Ggt).
Figure 4: This figure shows a graph where the percentage of infection in wheat plants is compared, in relation to the necrotic roots of infected wheat plants (+Ggt), grown from seeds not inoculated with the lyophilized bacterial consortium (Control); grown from seeds inoculated with each of the bacterial strains separately that are part of the consortium, that is, *Serratia sp.* 188.3 (Serratia); *Bacillus sp.* 4B (Bacillus); and *Acinetobacter sp.* E6.2 (Acinetobacter); and grown from seeds inoculated with the lyophilized bacterial consortium (Consortium);. The letters indicate statistical differences (the letters were obtained through the Tukey test), where different letters indicate statistical differences at p<0,05.

### Detailed description of the invention

The present invention discloses a bacterial consortium lyophilized, which comprises two strains of endophytic bacteria (*Acinetobacter sp.* and *Serratia sp.*) and a rhizospheric bacterial strain (*Bacillus sp*)*,* the method of obtaining the lyophilized bacterial consortium, and its use in the inhibition of the development, treatment and/or prevention of diseases caused by plant pathogens, as well as in the promotion of the growth of said plants. Likewise, a method is disclosed to inhibit the development of diseases caused by plant pathogens and promote their growth, which comprises applying the lyophilized bacterial consortium to seeds from which said plants grow.

The bacterial strains used in the present invention have been isolated and selected from suppressive soils, based on their plant growth promotion characteristics (based on a correlation dependent on their phosphorus solubilization capacity and siderophore production), to the production of phenazine-type antibiotics (by identifying the gene which codes for phenazine by PCR using specific primers) and mainly to the ability to inhibit the growth of the fungus *Gaeumannomyces graminis* var. *Tritici* (*Ggt*) *in vitro.*

The strains of the bacterial consortium of the present invention were deposited in the Chilean Collection of Microbial Genetic Resources (CChRGM), in its capacity as International Deposit Authority (IDA). The strain *Acinetobacter sp.* (E6.2) was deposited in the CChRGM under the Access No.: RGM 3033, dated August 11, 2020. The strain *Serratia sp.* (188.3) was deposited in the CChRGM, under the Access No.: RGM 2949, dated January 28, 2020. The strain *Bacillus sp.* (4B) was deposited in the CChRGM, under the Access No.: RGM 2948, dated January 28, 2020.

The strains of the bacterial consortium of the invention present one or more of the following characteristics: capacity for phosphorus solubilization, production of siderophores, production of phenazines and inhibition of *Ggt in vitro.*

Generally speaking, the strain *Acinetobacter sp.* E6.2 (Access No.: RGM 3033) has the ability to produce phenazines and significantly inhibit the development of *Ggt in vitro.* On the other hand, the strains of *Bacillus sp.* 4B (Access No.: RGM 2948) and *Serratia sp.* 188.3 (Access No.: RGM 2949) have the ability to solubilize phosphorus, produce siderophores and partially inhibit the development of *Ggt in vitro.*

The present invention discloses a lyophilized bacterial consortium comprising the strain of *Acinetobacter sp.* E6.2 (Access No.: RGM 3033), the strain of *Bacillus sp.* 4B (Access No.: RGM 2948) and the strain of *Serratia sp.* 188.3 (Access No.: RGM 2949).

The lyophilized bacterial consortium according to the present invention has 1 x10¹⁴ to 4×10¹⁶ CFU per gram of lyophilized bacterial consortium.

The lyophilized bacterial consortium according to the invention can be used to treat and/or prevent infectious diseases caused by plant pathogens and to promote plant growth. Likewise, the lyophilized bacterial consortium can be applied to seeds, in a method to inhibit the development of diseases caused by plant pathogens and promote plant growth.

Preferably, but not limited to, the lyophilized bacterial consortium according to the present invention can be used to treat and/or prevent plant diseases caused by the fungus *Gaeumannomyces graminis* var. *Tritici (Ggt).*

Preferably, but without being limited to, the lyophilized bacterial consortium according to the present invention can be used to treat diseases, preferably of grasses, more preferably of wheat, rye and triticale.

Likewise, the present invention discloses methods of preparation of the lyophilized bacterial consortium.

In one embodiment, the method of preparing the lyophilized bacterial consortium comprises the following steps:
i). inoculate a sterile culture medium with a bacterial strain of *Acinetobacter sp.,* with a bacterial strain of *Bacillus sp.,* and with a bacterial strain of *Serratia sp.* in the proportions 37 %, 27 % and 36 %, respectively, and incubate for 24 to 36 hours, with agitation between 50-150rpm, at a temperature of 28 °C, to obtain a bacterial broth;
ii).centrifuge the bacterial broth obtained in step (i) at between 4000-6000 rpm, for 10-15 minutes, to obtain a bacterial pellet;
iii).wash the bacterial pellet obtained in step (ii) at least 3 times with a wash solution;
iv).freeze the bacterial pellet obtained in step (iii) for 12 to 16 hours, at -50 to -80 °C, using a medium that contains a compound that prevents cell damage; and
v).lyophilize the pellet from step (iv) in a lyophilizer to obtain the lyophilized bacterial consortium.

In another embodiment, the preparation method of the lyophilized bacterial consortium can be done by producing and lyophilizing the bacterial strains separately, and later mixing them to obtain the lyophilized bacterial consortium. In said embodiment, the method for preparing the lyophilized bacterial consortium comprises the following steps:
i). inoculate a first sterile culture medium with a bacterial strain of *Acinetobacter sp.,* a second sterile culture medium with a bacterial strain of *Bacillus sp.,* and a third sterile culture medium with a bacterial strain of *Serratia sp..,* and incubate for 24 to 36 hours, with agitation between 50-150 rpm, at a temperature of 28 °C, to obtain a first, a second and a third bacterial broth, respectively;
ii). centrifuge the first, second and third bacterial broth obtained in step (i), respectively, at between 4000-6000 rpm, for 10-15 minutes, to obtain a first, second and third bacterial pellet, respectively;
iii). washing the first, second and third bacterial pellet obtained in step (ii), respectively, at least 3 times with a washing solution;
iv). freezing the first, second and third bacterial pellet obtained in step (iii), respectively, for 12 to 16 hours, at -50 to -80°C, using a medium containing a compound that prevents cell damage;
v). lyophilizing the first, second and third bacterial pellet obtained from step (iv), respectively, in a lyophilizer to obtain a first, second and third bacterial lyophilizate, wherein the first lyophilizate comprises the bacterial strain of *Acinetobacter sp.,* the second lyophilizate comprises the bacterial strain of *Bacillus sp.,* and the third lyophilizate contains the bacterial strain of *Serratia sp..;* and
vi). mix the first, second and third lyophilizate in the proportions 37%, 27% and 36%, respectively, to obtain the lyophilized bacterial consortium.

In this second embodiment, the only difference is found in the moment in which the bacterial strains are combined to form the bacterial consortium, being in step (i) of the method of the first embodiment described above, and in step vi) in the method of the second embodiment described above. However, one skilled in the art will understand that whichever method is used, the result in relation to the lyophilized bacterial consortium will be the same.

In connection with the embodiments described above, preferably the bacterial strain of *Acinetobacter sp.* is strain E6.2 (Access No.: RGM 3033), the bacterial strain of *Bacillus sp.* is strain 4B (Access No.: RGM 2948) and the bacterial strain of *Serratia sp.* is strain 188.3 (Access No.: RGM 2949).

If the bacterial strains are stored and not available in fresh culture prior to carrying out the method, a step prior to step (i) described above is carried out, in which said bacterial strains are reactivated by performing a pre-inoculum in a medium that allows adequate recovery of bacterial strains.

The bacterial strains can be stored through any conventional technique. Preferably, but not limited to, the stored bacterial strains are in a solution of 20 % glycerol and 80 % culture medium. Preferably, but not limited to, the culture medium is LB culture medium.

Preferably, but without limitation, the pre-inoculum of the bacterial strains is carried out in a medium that allows adequate recovery of the bacterial strains, diluting the bacterial strains 50 to 100 times in said culture medium. The culture medium that allows adequate recovery of the bacterial strains is selected from any general medium suitable for bacterial growth. Preferably, but without limitation, the general medium suitable for bacterial growth is selected from the group comprising: nutrient broth, malt extract, MRS medium, TSA medium and LB medium. More preferably, the culture medium that allows adequate recovery of the bacterial strains is LB medium.

For reactivation, preferably, the pre-inoculum is incubated for 24 to 36 hours, with agitation between 50-150 revolutions per minute (rpm) at a temperature of 28 °C.

In step (i) of the methods, the sterile culture medium is a culture medium suitable for the growth of the bacterial strains, which is selected from any general medium suitable for bacterial growth. Preferably, but without limitation, said general medium suitable forbacterial growth is selected from the group comprising: nutrient broth, malt extract, MRS medium, TSA medium and LB medium. More preferably, the culture medium suitable for the growth of the bacterial strains, i.e., the sterile culture medium, is LB medium.

Step (ii) of centrifugation of the methods is preferably carried out at room temperature.

The washing solution used in step (iii) of the methods can be distilled water or a saline solution, where the saline solution is prepared with salts selected from the group comprising: NaOH and KOH. Preferably, the saline solution is 0,5-1% (w/v) NaOH.

The compound used in step iv) of the methods, to avoid cell damage, is selected from the group comprising skimmed milk, semi-skimmed milk, whole milk, bovine serum, casein, polysorbate 20, polysorbate 80 and triton. Preferably, the compound that prevents cell damage is at a concentration between 10-20 % w/v.

The lyophilizing step (v) of the methods, preferably, but without limitation, it is carried out for two days at a temperature between -40 °C and -50 °C and at a pressure between 8-200 Pa (0,080 to 0,2 mBar). The lyophilization time will depend on the operational conditions. Preferably, but not limited to, the lyophilization time is 1-3 days.

Once the lyophilized bacterial consortium is obtained, the microbial load is determined by any conventional method. Preferably, the microbial load is determined by counting the colony-forming units (CFU) of the lyophilized material in LB culture medium, considering the dilutions and the initial weight of the powder.

This application also refers to the use of lyophilized bacterial consortium to inhibit the development prevent and/or treat diseases caused by plant pathogens and to promote plant growth. Preferably, the use of the lyophilized bacterial consortium serves to prevent the "take-all" disease (*Ggt*). More preferably, the use of the lyophilized bacterial consortium serves to prevent the "take-all" disease (*Ggt*) in cereal plants. Even more preferably, the use of the lyophilized bacterial consortium serves to inhibit the development of the pathogen or prevent the "take-all" disease (*Ggt*) in cereal plants, such as, but not limited to, wheat, rye, triticale.

This application also discloses a method to inhibit the development of infectious diseases caused by plant pathogens and promoting plant growth, which includes applying the lyophilized bacterial consortium to seeds from which said plants grow, to produce seeds inoculated with the bacterial consortium, wherein applying the lyophilized bacterial consortium to the seeds comprises the steps of:
(a) disinfect the seeds with a superficial disinfection method;
(b) mix the seeds obtained from step (a) with 10 -20 % by weight of a magnesium/calcium mixture (such as magnecal^{®}), 15% by weight of water and 0,2 % by weight of an adhesive glue mixture liquid and water (ratio 1:9);
(c) add a charge of 1×10⁹ to 1×10¹¹ CFU of the lyophilized bacterial consortium, obtained from any of the methods preparation of the lyophilized bacterial consortium described above, to the mixture of step (b);
(d) combine and homogenize the mixture resulting from step (c) at a speed between 50 and 100 rpm, for a period of 5 to 15 minutes at room temperature; and
(e) dry the seeds obtained in step (d) at a temperature between 15 to 25 °C for at least 3 hours, to obtain seeds inoculated with the bacterial consortium.

The superficial disinfection method of the seeds in step (a) can be any method that does not alter the subsequent germination of the seeds. Preferably, surface disinfection is carried out using a 15 % -20 % ethanol solution plus 1 % - 2 % sodium hypochlorite for 2-5 min, which is later rinsed.

Step (d) of combining and homogenizing the mixture is carried out at a low speed so that the seeds are impregnated with the mixture and to prevent them from sticking.

The seeds inoculated with the lyophilized bacterial consortium obtained from the previously described method can be used to be planted directly in the soil or stored for a prolonged period, at room temperature, avoiding humidity. Preferably, the seeds can be stored for a period of between 1 and 3 years.

### Examples

The following examples are merely illustrative and do not represent a limitation of the present invention in any way.

### Example 1: Characterization of the bacterial strains of the bacterial consortium

Initially, the strains of the bacterial consortium according to the present invention were characterized, that is, the strain of *Acinetobacter sp.* E6.2 (Access No.: RGM 3033), the strain of *Bacillus sp.* 4B (Access No.: RGM 2948) and the strain of *Serratia sp.* 188.3 (Access No.: RGM 2949).

The characterization was carried out specifically analyzing its ability to inhibit the development of the fungus *Ggt in vitro* and the traits of these strains that could be associated with characteristics of plant growth promoting bacteria (PGPB).

To evaluate the PGPB type traits, the phosphorus solubilization capacity and the siderophore production capacity of the different strains were analyzed.

The solubilization capacity of phosphorus in agar was verified according to the formation of a halo in NBRIP (National Botanical Research Institute' phosphate) medium supplemented with tricalcium phosphate (Ca₃[PO₄]₂). This medium contains (in g/L): Glucose,10; (NH₄)₂SO₄, 0,1; KCI, 0,2; MgSO₄×7H2O, 0,25; MgCl₂×6H2O, 5; Agar, 14; pH: 7 enriched with tricalcium phosphate, Ca₃PO4, 0,5% as a source of inorganic and insoluble phosphate. In addition, they were tested with PSM medium (phytase screening medium) which contains sodium phytate (C₆N₆Na₁₂O₂4P₆×H2O) as the sole source of phosphorus (5 g/L) and is composed of the following components per L, 10 g sucrose, 2 g (NH₄)2SO₄, 3 g tryptone, 2 g yeast extract, 0,5 g KCI, 0,5 g MgSO₄, 0,01 g MnSO4·5H₂O, 0,01 g FeSO₄, 1 g Triton X-100 and 15 g agar, pH: 7,0. Siderophore production was evaluated on agar plates with medium supplemented with chrome azurol S (CAS).

On the other hand, to assess the production capacity of antibiotics, it was analyzed the expression of the gene that encodes phenazines, performing a PCR assay using specific primers for the gene that encodes the antibiotic phenazine (PHZ1,5'-GGCGACATGGTCAACGG-3' and PHZ2, 5'-CGGCTGGCGGCGTATTC-3'), wherein said antibiotic was identified in the band that amplifies a region of 790 base pairs (bp). (Figure 1).

Finally, the percentage of inhibition of the development of *Ggt* that each of the strains had *in vitro* was analyzed. For the above, *Ggt* was cultivated on PDA agar plates at 25 °C for 1 week. Agar disks (4 mm in diameter) containing Ggtwere aseptically incised and transferred to the center of agar plates containing LB/PDA medium (1:1). Then, two drops (5 µL) of each bacterial suspension were taken and placed in two diametric positions 2 cm from the agar disk containing the inoculum of *Ggt.* The growth of fungal mycelia was measured after incubation for 2, 5 and 7 days at 25 °C in the dark. The inhibition percentage was calculated based on the growth of the fungus without bacterial presence (control).

As a summary, Table 1, shows the PGPB-type traits, that is, phosphorus solubilization and siderophore production, as well as the ability to produce phenazines and the ability to inhibit the pathogen *Ggt in vitro,* by each of the bacterial strains used in the present invention independently.

**Table 1. Individual characterization of the strains that make up the bacterial consortium**

| Strain | Phosphorus solubilization | Siderophore production | Phenazine production | % inhibition of *Ggt in vitro* |
|---|---|---|---|---|
| *Bacillus sp.* 4B | +++ | Positive | positive | 40 |
| *Serratia sp.* 188.3 | + | Positive | positive | 20 |
| *Acinetobacter sp.* E6.2 | negative | Negative | positive | 100 |

As shown in Table 1, in relation to the PGPB traits, which could be associated with the promotion of plant growth, the strain of *Acinetobacter sp.* E6.2 did not exhibit characteristics of PGPB, that is, phosphorus solubilization and siderophores production, but was only positive for the production of phenazine-type antibiotics. Through the PCR assay, it was also determined that the strains of *Bacillus sp.* 4B and *Serratia sp.* 188.3 were also capable of producing the antibiotic phenazine, since, as the strain of *Acinetobacter sp.* E6.2, they also showed a band that amplified a region of 790 bp consistent with the amplification of the gene that encodes said antibiotic (Figure 1).

Additionally, the strains of *Bacillus sp.* 4B and *Serratia sp.* 188.3 were able to solubilize phosphorus and produce siderophores, characteristics that, as previously mentioned, could be correlated with the ability of these strains to promote plant growth.

On the other hand, regarding the ability to inhibit the growth of *Ggt in vitro,* Table 1 shows that the strain of *Acinetobacter sp.* E6.2 inhibited 100% of the growth of the mycelium of *Ggt in vitro,* the strain of *Bacillus sp.* 4B inhibited the growth of mycelium *Ggt in vitro* by 40% and the strain of *Serratia sp.* 188.3 inhibited the growth of the mycelium *Ggt in vitro* by 20%.

Based on these results, the inventors considered that the mixture of the previously characterized isolated bacterial strains showed great potential for the control of *Ggt* and the promotion of plant growth.

### Example 2: Preparation of the bacterial consortium.

In the first place, compatibility tests were carried out between the strains, to verify that a consortium could be generated where the three bacterial strains of the invention were active. For this, a confrontational growth was carried out, measuring by optical density (at 600nm) the same bacterial quantity of the three isolates that make up the consortium and inoculating by streaking a Petri dish with solid LB medium, the three strains of the invention, that is, *Acinetobacter sp.* E6.2, *Bacillus sp.* 4B, and *Serratia sp.* 188.3. in different combinations. In particular, the following combinations were used: *Acinetobacter sp.x Bacillus sp., Acinetobacter sp.x Serratia sp.; Bacillus sp.* X *Serratia sp.* and *Acinetobacter sp.x Bacillus sp. x Serratia sp.,* to demonstrate optimal growth of the three strains. From this assay, optimal growth was determined and it was found that the strains of the invention can indeed grow in consortium without inhibiting each other's growth.

With this background, for the preparation of the consortium, samples of each of the bacterial strains previously stored in a glycerol stock (20 % glycerol 80 % LB culture medium) were used.

To reactivate the bacterial strains of the invention, that is bacteria of the strain of *Acinetobacter* sp E6.2 (Access No.: RGM 3033), of *Serratia* sp 188.3 (Access No.: RGM 2949) and of *Bacillus* sp 4B (Access No.: RGM 2948), a pre-inoculum was prepared, where 50 µL of each microorganism were taken and added to a tube with 5 mL of LB medium. This pre-inoculum was incubated for 24 hours at 100 rpm and 28 °C.

From the previously described pre-inoculum, an inoculum was prepared by adding the strains of *Acinetobacter sp., Bacillus sp.* and *Serratia sp.,* in a proportion of 37 %, 27 % and 36 %, respectively, to a vial containing one liter of LB medium, in order to use the same proportion of microorganisms, which was calculated based on the optimal growth previously determined. This culture was incubated for 24 hours, at 100 rpm and 28 °C.

Subsequently, the bacteria were centrifuged at 5000 rpm for 10 minutes at room temperature. The obtained bacterial pellet was washed three times with a 0,85 % w/v NaOH saline solution.

Once washed, the bacterial pellet was frozen at -80 °C in a 10 % skim milk solution overnight. Subsequently, the lyophilization process was carried out for two days at a temperature of -52 °C.

Once the lyophilized bacterial consortium was obtained, the lyophilizate was counted, after which the value obtained corresponded to 3,3×10¹⁶ CFU per gram of lyophilizate.

### Example 3: Preparation of seeds inoculated with the lyophilized bacterial consortium.

The previously obtained lyophilized bacterial consortium was used to be applied to wheat seeds.

For this, 1000 g of wheat seeds were first disinfected with a 20 % ethanol solution plus 1 % sodium hypochlorite for 5 min. The seeds were rinsed with running water and then mixed with 100 g of magnecal^{®}, 150 mL of water and 20 mL of a mixture of liquid adhesive glue (cold glue) and water (1:9 ratio).

Using the count value of the lyophilized product from example 2, it was estimated that for each kilogram of seed, 0,5 grams of bacterial lyophilized product should be added, to reach a microbial load of 1×10¹¹ CFU.

Based on this, 0,05 g of the lyophilized bacterial consortium was added to the seed mixture to obtain a load of 1×10¹⁰ CFU. Then, the previous mixture was homogenized at a speed of 20 rpm, for a period of 10 minutes, at room temperature, so that the seeds were impregnated with the mixture. Finally, the seeds inoculated with the bacterial consortium were dried at room temperature for at least 3 hours.

The same procedure was carried out using each of the strains of the bacterial consortium independently (instead of the consortium), in order to produce seeds inoculated with only one of the strains to verify the particular behavior of each one of them in the inhibition of the growth of *Ggt* in example 4.

### Example 4: Effect of the lyophilized bacterial consortium on the growth of wheat and on the inhibition of the development of Ggt in wheat.

To carry out the tests, firstly, the fungus *Gaeumannomyces graminis* var. *Tritici (Ggt)* was grown for 7 days in potato dextrose agar (PDA) medium at a temperature of 25 °C for 5 to 7 days. Subsequently, the fungus was inoculated into sterile oats as a substrate, which had been sterilized in an autoclave at 121 ° C for three consecutive days. After 20 days, the oats with the pathogen were collected, ground, and used as inoculum for soil infection. For this, the soil was inoculated at 1 % (1 gram of infection inoculum per 100 grams of soil), where this concentration is equal to or higher than that found naturally in infected soils.

To evaluate the effect of the lyophilized bacterial consortium on the growth of wheat and on the inhibition of the development of *Ggt* in wheat, four experimental conditions were analyzed. In the first one, uninoculated wheat seeds were sown in pots using the infected soil described above (Control+Ggt). In the second, wheat seeds inoculated with the lyophilized bacterial consortium obtained in Example 3 were sown in pots using the infected soil described above (Consortium+Ggt). In the third, uninoculated wheat seeds were sown in pots using the uninfected soil (Control -Ggt). Finally, in the fourth, wheat seeds inoculated with the lyophilized bacterial consortium obtained in example 3 were sown in pots using uninfected soil (Consortium -Ggt).

After 60 days under greenhouse conditions, the percentage of infection was determined by measuring root necrosis (black roots in relation to healthy roots), results that are shown in Figure 2 and in Table 2 below:

**Table 2. Effect of lyophilized bacterial consortium on the percentage of Ggt infection in wheat plants**

| Condition | Infection (%) | Standard error |
|---|---|---|
| Control+Ggt | 11,8342008 | 1,35173522 |
| Consortium+Ggt | 2,0424333 | 0,75375549 |
| Control -Ggt | 0,30737705 | 0,27492639 |
| Consortium-Ggt | 0,28195489 | 0,25218812 |

As shown in Table 2 and Figure 2, it can be observed that for the plants from seeds inoculated with the lyophilized bacterial consortium the infection of the phytopathogen (Consortium+Ggt) decreased by 80% in relation to the control of the plants from of uninoculated seeds (Control+Ggt). In addition, it can be observed that the treatments in which the phytopathogen has not been applied ("Control-Ggt" and "Consortium-Ggt") also present a small percentage of necrosis that could be associated with any stress factor. Consequently, from the results of Figure 2, it is possible to conclude that the lyophilized bacterial consortium of the present invention has the ability to inhibit the development of the fungus. *Gaeumannomyces graminis* var. *Tritici (Ggt)* in wheat plants.

Additionally, to evaluate the growth of the wheat plants, the biomass of the root and that of the leaves (aerial part) were weighed and the growth was calculated through the root biomass/aerial part biomass ratio, using the same conditions described above, that is, using seeds inoculated or not inoculated with the consortium and grown in the presence or absence of the *Ggt* pathogen. The results are set forth in Figure 3 and in Table 3 below:

**Table 3: Effect of lyophilized bacterial consortium on the growth of wheat plants.**

| Condition | Biomass ratio (root/aerial part) | Standard error |
|---|---|---|
| Control+Ggt | 0,26508572 | 0,02797187 |
| Consortium+Ggt | 0,41825243 | 0,06268516 |
| Control -Ggt | 0,55301984 | 0,08012779 |
| Consortium-Ggt | 0,74831961 | 0,09044248 |

Surprisingly, the inventors found that the consortium not only inhibited the development of *Ggt,* but also observed that the lyophilized bacterial consortium is capable of increasing biomass by more than 37% in the case of plants infected with the pathogen from seeds inoculated with the consortium (Consortium+Ggt) in relation to plants infected with the pathogen from non-inoculated seeds (Control+Ggt). Likewise, the lyophilized bacterial consortium increased biomass by more than 27% in the case of plants grown without the presence of the pathogen from seeds inoculated with the consortium (Consortium -Ggt) in relation to control plants from non-inoculated seeds (Control-Ggt), thus demonstrating that the lyophilized bacterial consortium according to the present invention would exhibit plant growth promoting properties (Figure 3).

Based on the results described above, it is shown that the proposed lyophilized bacterial consortium allows reducing the incidence of the *Ggt* pathogen and increase the productivity, in terms of biomass produced, of cereal plants, as in the example of treated wheat seeds.

The expert will be able to note that the choice of wheat plants was made only by way of example, since the method could be applicable to other cereal plants.

Finally, to evaluate the effect of each of the strains of the bacterial consortium independently, in comparison with the effect of the lyophilized bacterial consortium, on the inhibition of the development of *Ggt* in wheat plants, five additional experimental conditions were tested. In the first one, uninoculated wheat seeds were sown in pots using the infected soil described above (Control+Ggt). In the second, wheat seeds inoculated with the bacterial strain of *Serratia* sp 188.3 (Access No.: RGM 2949) obtained in example 3 were planted in pots using the infected soil described above (Serratia+Ggt). In the third, wheat seeds inoculated with the bacterial strain of *Bacillus sp* 4B (Access No.: RGM 2948), obtained in example 3 were planted in pots using the infected soil described above (Bacillus+Ggt). In the fourth, wheat seeds inoculated with the bacterial strain of *Acinetobacter* sp E6.2 (Access No.: RGM 3033), obtained in example 3 were planted in pots using the infected soil described above (Acinetobacter+Ggt). Finally, in the fifth, wheat seeds inoculated with the lyophilized bacterial consortium obtained in Example 3 were sown in pots using the infected soil described above (Consortium+Ggt). The results are shown in Figure 4 and in Table 4, which is shown below:

**Table 4. Effect of the lyophilized bacterial consortium and of each of the bacterial strains that comprise it on the percentage of Ggt infection in wheat plants**

| Condition | Infection (%) | Standard error |
|---|---|---|
| Control+Ggt | 11,83420076 | 1,351735221 |
| Serratia+Ggt | 6,021807354 | 2,236201839 |
| Bacillus+Ggt | 4,390924461 | 0,75531769 |
| Acinetobacter+Ggt | 4,130536695 | 0,78456296 |
| Ggt+Consortium | 2,042433299 | 0,753755489 |

From said results, it is evident that the lyophilized bacterial consortium according to the invention has a greater inhibitory effect on the growth of the *Ggt* fungus, that is, it has a significantly lower percentage of infection, at least 50% lower, than each of the strains alone, evidencing a cooperative effect between the bacterial strains. Consequently, it is possible to note that the consortium, due to its composition of the three specific bacterial strains used, gives the invention a technical advantage in terms of a high capacity to inhibit the development of the *Ggt* pathogen in field crops.

## Claims

1. Lyophilized Bacterial Consortium inhibitor of the development of diseases caused by plant pathogens and promoter of their growth, **CHARACTERIZED in that** it includes the following bacterial strains:
- a strain of *Acinetobacter sp.* and a strain of *Serratia sp.,* both endophytic strains; and
- a strain of *Bacillus sp* which is a rhizospheric strain,
wherein the strain of *Acinetobacter sp.* is the strain deposited in the Chilean Collection of Microbial Genetic Resources (CChRGM) with the Access No. RGM 3033; the strain of *Serratia sp.* is the strain deposited in the CChRGM under Access No. RGM 2949; and the strain of *Bacillus sp* is the strain deposited in the CChRGM under Access No. RGM 2948.

2. Lyophilized bacterial consortium according to claim 1, **CHARACTERIZED in that** it has 1 ×10¹⁴ to 4×10¹⁶ CFU per gram of lyophilized bacterial consortium.

3. Bacterial strain isolated from *Acinetobactersp* inhibitor of the development of diseases caused by plant pathogens and promoter of their growth, **CHARACTERIZED in that** the strain of *Acinetobacter sp* is the strain deposited in the CChRGM under Access No. RGM 3033.

4. Bacterial strain isolated from *Serratia sp* inhibitor of the development of diseases caused by plant pathogens and promoter of their growth, **CHARACTERIZED in that** the strain of *Serratia sp* is the strain deposited in the CChRGM under Access No. RGM 2949.

5. Bacterial strain isolated from *Bacillus sp* inhibitor of the development of diseases caused by plant pathogens and promoter of their growth, **CHARACTERIZED in that** the strain of *Bacillus sp* is the strain deposited in the CChRGM under Access No. RGM 2948.

6. Method for obtaining the lyophilized bacterial consortium according to any of claims 1-2, **CHARACTERIZED in that** it comprises the following steps:
i. inoculate a sterile culture medium with a bacterial strain of *Acinetobacter sp.,* with a bacterial strain of *Bacillus sp.,* and with a bacterial strain of *Serratia sp..,* in the proportions 37 %, 27 % and 36 %, respectively, and incubate for 24 to 36 h, with agitation between 50-150 rpm. at a temperature of 28 °C, to obtain a bacterial broth;
ii. centrifuge the bacterial broth obtained in step (i) at between 4000-6000 rpm for 10-15 minutes, to obtain a bacterial pellet;
iii. washing the bacterial pellet obtained from step (ii) at least 3 times with a washing solution;
iv. freeze the pellet from step (iii) for 12 to 16 hours at 50 to 80 °C, using a medium containing a selected compound that prevents cell damage; and
v. lyophilize the pellet from step (iv) in a lyophilizer to obtain the lyophilized bacterial consortium.

7. Method for obtaining the lyophilized bacterial consortium according to any of claims 1-2, **CHARACTERIZED in that** it comprises the following steps:
i). inoculate a first sterile culture medium with a bacterial strain of *Acinetobacter sp.,* a second sterile culture medium with a bacterial strain of *Bacillus sp.,* and a third sterile culture medium with a bacterial strain of *Serratia sp..,* and incubate for 24 to 36 hours, with shaking between 50-150 rpm at a temperature of 28 °C, to obtain a first, a second and a third bacterial broth, respectively;
ii). centrifuge the first, second and third bacterial broth obtained in step (i), respectively, at between 4000-6000 rpm, for 10-15 minutes, to obtain a first, second and third bacterial pellet, respectively;
iii). washing the first, second and third bacterial pellet obtained in step (ii), respectively, at least 3 times with a washing solution;
iv). freezing the first, second and third bacterial pellet obtained in step (iii), respectively, for 12 to 16 hours, at -50 to -80 °C, using a medium containing a compound that prevents cell damage;
v). lyophilizing the first, second and third bacterial pellet obtained from step (iv), respectively, in a lyophilizer to obtain a first, second and third bacterial lyophilizate, wherein the first lyophilizate comprises the bacterial strain of *Acinetobacter sp.,* the second lyophilizate comprises the bacterial strain of *Bacillus sp.,* and the third lyophilizate includes the bacterial strain of *Serratia sp..;* and
vi). mix the first lyophilizate, the second lyophilizate and the third lyophilizate in the proportions 37 %, 27 % and 36 %, respectively, to obtain the lyophilized bacterial consortium.

8. The method according to claim 6 or 7, **CHARACTERIZED in that** it additionally comprises a step prior to step (i), where the bacterial strains are reactivated, when they are stored, performing a pre-inoculum with said bacterial strains in a sterile culture medium.

9. The method according to claim 8, **CHARACTERIZED in that** the bacterial strains, when stored, are in a solution of 20% glycerol and 80% culture medium.

10. The method according to any of claims 8 or 9, **CHARACTERIZED in that** the preinoculum is carried out by inoculating the bacterial strains diluted 50 to 100 times in a sterile culture medium.

11. The method according to claim 10, **CHARACTERIZED in that** the preinoculum is incubated for 24 to 36 hours with agitation between 50-150 rpm at a temperature of 28 °C.

12. The method according to any of claims 6 to 11, **CHARACTERIZED in that** the bacterial strain of *Acinetobacter* sp. is strain E6.2 (Access No.: RGM 3033), the bacterial strain of *Bacillus sp.* is strain 4B (Access No.: RGM 2948) and the bacterial strain of *Serratia* sp. is strain 188.3 (Access No.: RGM 2949).

13. The method according to any of claims 6 to 12, **CHARACTERIZED in that** the sterile culture medium is selected from the group comprising: nutritive broth, malt extract, MRS medium, TSA medium and LB medium.

14. The method according to claim 13, **CHARACTERIZED in that** the sterile culture medium is LB medium.

15. The method according to any of claims 6 to 14, **CHARACTERIZED in that** step (ii) of centrifugation is carried out at room temperature.

16. The method according to any of claims 6 to 15, **CHARACTERIZED in that** the wash solution of step (iii) is distilled water or a saline solution.

17. The method according to claim 16, **CHARACTERIZED in that** the saline solution is selected from the group that comprises a solution of NaOH and a KOH solution.

18. The method according to claim 17, **CHARACTERIZED in that** the saline solution is 0,5-1 % NaOH.

19. The method according to any of claims 6 to 18, **CHARACTERIZED in that** the compound that prevents cell damage, which is used in step (iv), is selected from the group comprising: skimmed milk, semi-skimmed milk, whole milk, bovine serum, casein, polysorbate 20, polysorbate 80 and triton.

20. The method according to claim 19, **CHARACTERIZED in that** the compound that prevents cell damage is at a concentration between 10-20 % w/v.

21. The method according to any of claims 6 to 20, **CHARACTERIZED in that** the lyophilization of step (v) is carried out for two days, at a temperature of -40 °C to -60 °C and a pressure of 8-200 Pa (0,080 to 2 mBar).

22. Use of the bacterial consortium according to any of claims 1-2, **CHARACTERIZED in that** serves to inhibit the development prevent and/or treat diseases caused by plant pathogens and to promote plant growth.

23. The use according to claim 22, **CHARACTERIZED in that** the infectious disease is caused by the plant pathogen *Gaeumannomyces graminis* var. *Tritici (Ggt).*

24. The use according to any of claims 22-23, **CHARACTERIZED in that** the plant is a cereal.

25. The use according to claim 24, **CHARACTERIZED in that** the cereal is selected from the group comprising: wheat, rye and triticale.

26. Method for inhibiting the development of diseases caused by plant pathogens and promoting plant growth, **CHARACTERIZED in that** it includes applying the lyophilized bacterial consortium according to any of claims 1-2 to seeds from which said plants grow, to produce seeds inoculated with the bacterial consortium, wherein the application of the lyophilized bacterial consortium in the seeds comprises the steps of:
(a) disinfect the seeds with a superficial disinfection method;
(b) mix the seeds obtained in step (a) with 10 - 20% by weight of a magnesium/calcium mixture, 15% by weight of water and 0,2% by weight of a mixture of liquid adhesive glue and water (ratio 1:9);
(c) add a charge of 1×10⁹ to 1×10¹¹ CFU of said lyophilized bacterial consortium, to the mixture of step (b);
(d) combine and homogenize the mixture resulting from step (c) at a speed between 50 and 100 rpm, for a period of 5 to 15 minutes at room temperature; and
(e) dry the seeds obtained in step (d) at a temperature between 15 and 25 °C for at least 3 hours, to obtain seeds inoculated with the bacterial consortium.

27. The method according to claim 26, **CHARACTERIZED in that** the superficial disinfection method of the seeds in step (a) consists of washing the seeds with a solution of 15 % - 20 % ethanol plus 1% -2% hypochlorite of sodium, for 2-5 min; and rinse the seeds.

28. The method according to any of claims 26-27, **CHARACTERIZED in that**, optionally, the inoculated seeds are stored at room temperature.
